# EUROPEAN PATENT APPLICATION

(11) **EP 2 050 747 A1**
(43) Date of publication of application: **22.04.2009**
(21) Application number: 07291250.4
(22) Date of filing: 12.10.2007
(51) Int. Cl.: C07D 471/04, A61K 31/437, A61P 35/00

(54) **Dimers of harmol or of its derivatives and uses thereof**

(71) Applicant: BioAlliance Pharma, 75015 Paris (FR); ECOLE NORMALE SUPERIEURE DE CACHAN, 94235 Cachan (FR); CENTRE NATIONAL DE LA RECHERCHE SCIENTIFIQUE (CNRS), 75016 Paris (FR); Universite De Paris 11-Paris Sud, 91405 Orsay (FR)
(72) Inventor: Desmaële, Didier, 94260 Fresnes (FR); Auclair, Christian, 75016 Paris (FR); Zouhiri, Fatima, 92290 Chatenay-Malabry (FR); Polard, Valérie, 91700 Sainte-Genevieve des Bois (FR); Maksimenko, Andréi, 97800 Brunoy (FR)
(74) Representative: Colombet, Alain André

(57) **Abstract**

The present invention relates to a compound of formula (II):

A-X-A (II)

wherein:
- A represents in particular a radical of formula (1): 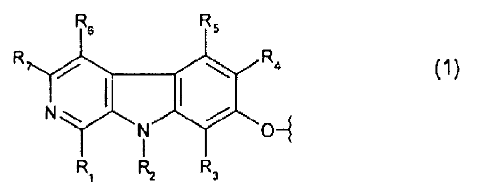 wherein R₁ represents in particular a methyl group, R₂ represents in particular an alkyl group of 1 to 10 carbon atoms, and R₃ to R₇ represent preferably H,
- X represents in particular an alkyl radical having 1 to 10 carbon atoms for the treatment of cancer.

## Description

This invention relates to the syntheses of dimers of harmol or of its derivatives such as harmine and β-carbolines, and to the use of such dimers for the treatment of cancer.

Cancer is a disease of humans and animals which is for the most part fatal and which is caused by the uncontrolled growth of endogenous cells.

Aside from the, if possible radical, surgical removal of the tumor, the possibilities for treating malignant tumors include radiological therapy, immunotherapy and chemotherapy. The substances of chemotherapy interfere specifically in certain processes in cell division. Different cytostatic drugs have been developed. However, due to massive side-effects, all these agents suffer from severe disadvantages, such that the death of the affected patient is only delayed and not averted. Furthermore, the degenerate (cancer) cells develop resistances to the agents which are used; while the medicaments which are being used at the time then no longer have any cytostatic effect, they are still toxic as a consequence of the side-effects. In addition, it has been found that the efficacy achieved by using cytostatic agents in combination or in sequence exceeds that achieved by using a single cytostatic agent (monotherapy) and, as a result, it is possible that the substantial side-effects are not additive in connection with polychemotherapy.

In non cancer cells, adhesion to the extracellular matrix and to neighbouring cells plays a central role in the control of cell survival, growth, differentiation and motility (K.A. Beningo et al., J. Cell Biol. 153 (2001), pp. 881-888; S.M. Frisch and R.A. Screaton, Curr. Opin. Cell Biol. 13 (2001), pp. 555-562 and F.M. Watt, EMBO J. 21 (2002), pp. 3919-3926). Upon oncogenic transformation, profound changes occur in cell morphology and the organization of the cytoskeleton, in cell motility and in growth factor- or adhesion-dependent cell proliferation (for a review, see G. Pawlak and D.M. Helfman, Curr. Opin. Genet. Dev. 11 (2001), pp. 41-47). Disruption of the actin cytoskeleton and a concomitant reduction in the number of focal adhesions are common features accompanying cell transformation induced by various oncogenes. That the actin cytoskeleton plays a fundamental role in oncogenesis is suggested by the association of anchorage-independent growth and tumorigenicity with the rearrangements of the actin filament network observed in transformed cells (P. Kahn et al., Cytogenet. Cell Genet. 36 (1983), pp. 605-611). Adhesive interactions involve specialized transmembrane receptors that are linked to the cytoskeleton through junctional plaque proteins (for a review, see Nagafuchi, Curr. Opin. Cell Biol. 13 (2001), pp. 600-603). The synthesis of several actin-binding proteins, including α-actinin, vinculin, tropomyosin and profilin, is down-regulated in transformed cells and overexpressing these proteins in tumor cells suppresses the transformed phenotype, which allows them to be considered as tumor suppressors.

Harmine is a natural plant alkaloid product which was isolated from the Peganum harmala seeds. Peganum harmala (Zygophyllaceae) is a plant widely distributed in semi arid rangelands in the Central Asia, North Africa, Middle East and Australia. The pharmacologically active compounds of P.harmala are several alkaloids that are found especially in the seeds and the roots. These incude β-carbolines such as harmine, harmaline, harmol, harmalol and harman, and quinazoline derivatives: vasicine and vasicinone.

Peganum harmala alkaloids were found to possess significant antitumour potential (Lamchouri and al., Therapie, 1999, 54(6):753-8). Proliferation of tumoral cells lines was significantly reduced at all tested concentrations (20-120 micrograms/ml) during the first 24 h of contact. A cell lysis effect occurred after 24 h and increased thereafter to complete cell death within 48-72 h, depending on tested concentration (Lamchouri and al, Fitoterapia, 2000, 71(1):50-4).

Harmine was reported to exhibit strong cytotoxicity against a number of human tumor cell lines (Ishida and al, Bioorg Mad Chem Lett, 1999, 9(23):3319-24).

Furthermore it was shown that harmine is a selective inhibitor of cyclin-dependent kinases (CDKs) that regulate the cell division cycle and cell proliferation (Song et al., Biochem Biophys Res Commun., 2004. 317(1):128-32).

The degree of aromaticity of the tricyclic ring and the positioning of substituents of harmine are important for inhibitory CDKs activity (Song et al., Bioorg Med Chem Lett, 2002, 12(7):1129-32)

However, histologic study showed experimental toxicity of harmine on the central nervous system (CNS) (Lamchouri and al, Ann Pharm Fr, 2002, 60(2):123-9)

Furthermore, *in vivo* in mice, despite an antitumor effect, an acute toxicity was described: tremble, twitch and jumping (Qi Chen and al, International Journal of cancer, 2005).

It was demonstrated that harmine is monoamine oxydase inhibitor and has hallucinogenic properties.

For all these reasons, the development of drugs effective against human cancers and having limited toxic side effects remains a critical need. The challenge is in particular to succeed in identifying anticancer drugs acting mainly through a non-cytotoxic process and with limited toxic side effect.

The inventors hypothesized to identify compound able to transform tumour cell phenotype with less cytotoxic effect with as original target an actin chain as the inventors have demonstrated that changes in cell phenotype, and more specifically in the cytoskeletal architecture, which is one of the main molecular mechanisms underlying tumor progression, could be a pertinent target process to identify new active anticancer drugs (WO2004/057337).

The inventors have unexpectedly demonstrated that harmol dimers have anticancer activity which is mediated by a non-cytotoxic process. The inventors have shown that harmol dimers increase actin network rearrangement, thereby inducing phenotypic reversion of tumor cells thanks to the rescue of adhesion and motility control, and are less cytotoxic than harmine. Moreover, phenotypic reversion is obtained with non-cytotoxic concentrations, i.e. concentrations which have no significant effect on both cell proliferation and cell survival.

Thus, it has now been found that harmol dimers provide anticancer drugs with a better efficacy than harmine acting mainly through a non-cytotoxic process and are consequently suitable to be used for the treatment and/or prophylaxis of cancer diseases. Surprisingly, the harmol dimers tested orally are active without neurotoxicity. The new actin chain target concept identified with harmol dimers is pharmacologically with less cytotoxic activity.

The compound identified by the inventors as inducing malignant phenotypic reversion at non-cytotoxic concentrations is a dimer of formula (II):

A-X-A (II)

wherein:
- A represents:
   - a radical of formula (1): 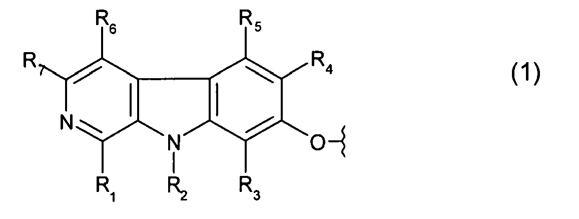
wherein:
* R₁ is chosen from the group consisting of:
   - an alkyl group of 1 to 20 carbon atoms, in particular a methyl group,
   - a COOR group, wherein R represents an alkyl group of 1 to 20 carbon atoms, and
   - a COR group, wherein R represents an alkyl group of 1 to 20 carbon atoms, preferably a methyl group,
* R₂ is chosen from the group consisting of:
   . H,
   . an alkyl group of 1 to 20 carbon atoms,
   . an aryl group of 6 to 30 carbon atoms, in particular a benzyl group, if appropriate substituted with a halogen such as F, said aryl group being preferably p-fluorobenzyl,
   . a COOR group, wherein R represents H or an alkyl group of 1 to 20 carbon atoms, R being in particular a t-butyl group, and
   . a COR group, wherein R represents an alkyl group of 1 to 20 carbon atoms, preferably a methyl group,
   . a SO₂Ar group, wherein Ar represents an aryl group of 6 to 30 carbon atoms,
* R₃, R₅, R₆, and R₇ represent, independently from each other, a group selected from:
   . H,
   . an alkyl group of 1 to 20 carbon atoms, and
   . an aryl group of 6 to 30 carbon atoms, in particular a benzyl group, if appropriate substituted with a halogen,
* R₄ represent, independently from each other, a group selected from:
   . H,
   . an alkyl group of 1 to 20 carbon atoms,
   . an aryl group of 6 to 30 carbon atoms, in particular a benzyl group, if appropriate substituted with a halogen,
   . NO₂, and
   . a NRR' group, wherein R and R' represent, independently from each other, H or an alkyl group of 1 to 20 carbon atoms,
      - or a radical of formula (2): 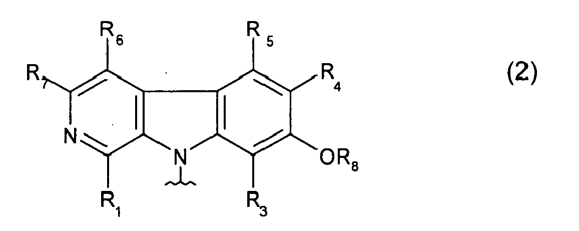
wherein:
* R₁, R₃, R₄, R₅, R₆, and R₇ are as defined above for formula (1),
* R₈ represents a group selected from:
   . H,
   . an alkyl group of 1 to 20 carbon atoms, and
   . a COR group, wherein R represents an alkyl group of 1 to 20 carbon atoms,
      - or a radical of formula (3): 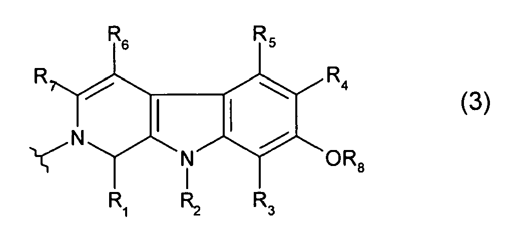
wherein R₁ to R₈ are as defined above for formula (2),
- X represents a saturated or unsaturated alkyl radical having 1 to 20 carbon atoms, eventually substituted with at least 1 oxygen atom, and preferably with at least 2 oxygen atoms, or a radical having the formula -(CH₂CH₂O)ₙCH₂CH₂- where n is a whole number ranging from 1 to 10, said alkyl radical being eventually substituted with at least one hydroxyl group, in particular with two hydroxyl groups, eventually engaged in a 5-membered ring to form a 1,3-dioxolane cycle, which is if appropriate substituted with at least one alkyl group of 1 to 10 carbon atoms, said 5-membered ring being in particular 2,2-dimethyl-1,3-dioxolane.

According to an embodiment, the dimers of the invention have the formula (III): 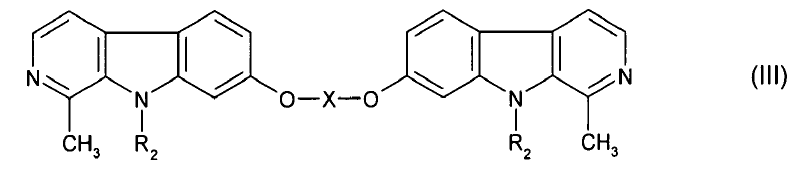 wherein:
- X is a saturated or unsaturated alkyl radical having 1 to 20, preferably 3 to 10, carbon atoms, or a radical having the formula -(CH₂CH₂O)ₙCH₂CH₂-, where n is a whole number ranging from 1 to 10,
   said alkyl radical being if appropriate substituted with at least one carbonyl, or peptidic bond, or at least one oxygen atom, or with at least one hydroxyl group, in particular with two hydroxyl groups, said hydroxyl groups being eventually engaged in a 5-membered ring to form a 1,3-dioxolane cycle, which is if appropriate substituted with at least one alkyl group of 1 to 10 carbon atoms, said 5-membered ring being in particular 2,2-dimethyl-1,3-dioxolane, and
- R₂ is as defined above.

According to a particular embodiment, the dimers of the invention have the formula (III) as mentioned above wherein X is selected from the group consisting of the following groups:
- a group of formula (3):

   -(CH₂)ₙ- (3)

   n being an integer varying from 1 to 10, and preferably equal to 3, 4, 5, or 6,
- a group of formula (4):

   -(CH₂)ₘ-O-(CH₂)ₚ-O-(CH₂)_{q}- (4)

   m, p, and q being integers varying from 1 to 4, and preferably equal to 2,
- a group of formula (5): 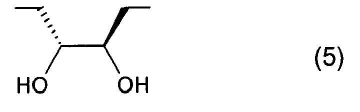
- a group of formula (6):

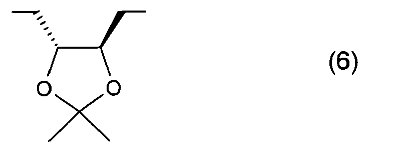

According to another embodiment, the dimers of the invention have the formula (V-1): 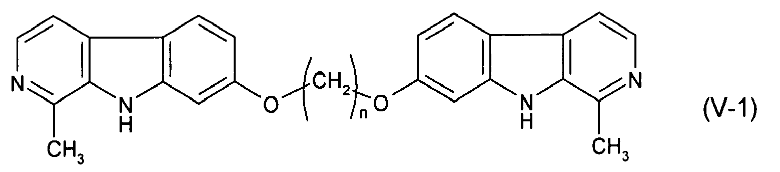 n being an integer varying from 1 to 10, and preferably equal to 3, 4, 5, or 6.

According to an embodiment, the harmol dimers of the invention have the formula (IV): 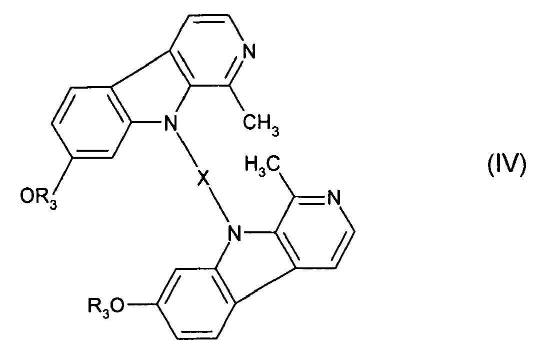 wherein:
- X is an alkyl radical having 2 to 10, preferably 3 to 5, carbon atoms, and
- R₃ is as defined above.

According to a particular embodiment, the harmol dimers of the invention have the formula (VIII-1): 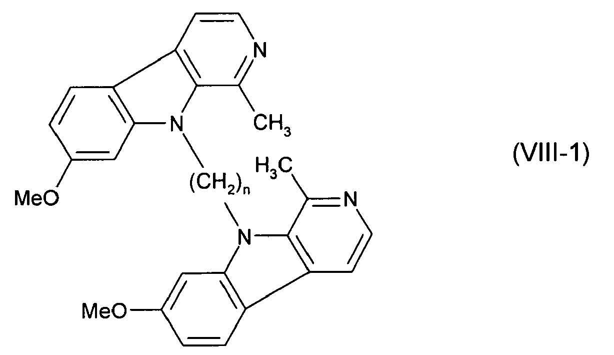 n varying from 2 to 10, preferably from 3 to 5.

The present invention furthermore relates to all obvious chemical equivalents of the compounds of the formula (I) according to the invention. These equivalents are compounds which exhibit only a slight chemical difference, and have the same pharmacological effect, or which are converted into the compounds according to the invention under mild conditions. Said equivalents also include, for example, pharmaceutically acceptable salts, reduction products, oxidation products, esters, ethers, acetals or amides of the compounds of the formula (II) as well as equivalents which the skilled person can prepare using standard methods and, in addition to this, all the optical antipodes and diastereomers and all the stereoisomeric forms.

As used herein, "dimer" means a molecule composed of two identical subunits linked together. The molecules in a dimer are connected with a covalent bond or covalent link (X).

The term "alkyl" means a saturated or unsaturated aliphatic hydrocarbon group which may be straight or branched having about 1 to about 20 carbon atoms in the chain. Preferred alkyl groups have 1 to about 12 carbon atoms in the chain, still preferably 1 to 6 carbon atoms. Branched means that one or lower alkyl groups such as methyl, ethyl or propyl are attached to a linear alkyl chain. «Lower alkyl» means about 1 to about 4 carbon atoms in the chain which may be straight or branched. The alkyl may be substituted with one or more «alkyl group substituants» which may be the same or different, and include for instance halo, cycloalkyl, hydroxy, alkoxy, amino, acylamino, aroylamino, carboxy.

"Pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

The term "pharmaceutically acceptable salt" refers to salts which retain the biological effectiveness and properties of the compounds of this invention and which are not biologically or otherwise undesirable. In many cases, the compounds of this invention are capable of forming acid and/or base salts by virtue of the presence of amino and/or carboxyl groups or groups similar thereto. Pharmaceutically acceptable acid addition salts may be prepared from inorganic and organic acids, while pharmaceutically acceptable base addition salts can be prepared from inorganic and organic bases. For a review of pharmaceutically acceptable salts see Berge, et al. ((1977) J. Pharm. Sd, vol. 66, 1). The expression "non-toxic pharmaceutically acceptable salts" refers to non-toxic salts formed with nontoxic, pharmaceutically acceptable inorganic or organic acids or inorganic or organic bases. For example, the salts include those derived from inorganic acids such as hydrochloric, hydrobromic, sulfuric, sulfamic, phosphoric, nitric, and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicyclic, sulfanilic, fumaric, methanesulfonic, and toluenesulfonic acid and the like.

More specifically, for the use of the invention, preferred dimers are:
- 7,7'-[Propane-1,3-diylbis(oxy)] bis(1-methyl-9H-β-carboline) of formula (V) 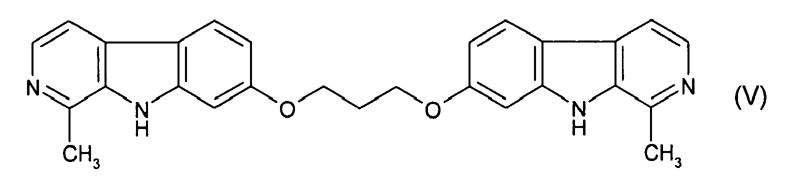
- 7,7'-[Pentane-1,5-diylbis(oxy)]bis(1-methyl-9H-β-carboline) of formula (VI): 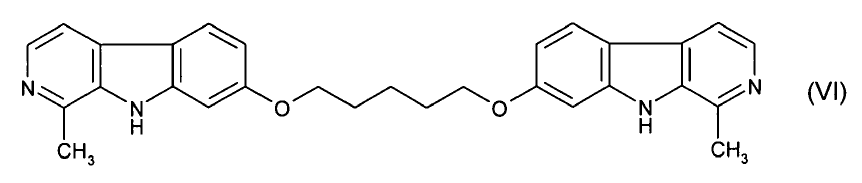
- 7,7'-[(2,2-dimethyl-1,3-dioxolane-4,5-diyl)bis(methyleneoxy)]bis(1-methyl-9H-β-carboline) of formula (VII) 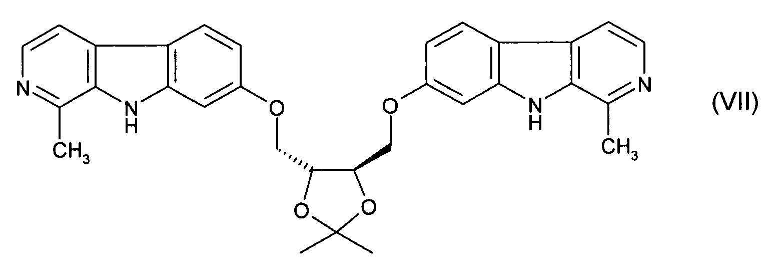
- 9,9'-pentane-1,5-diylbis(7-methoxy-1-methyl-9H-β-carboline) of formula (VIII) 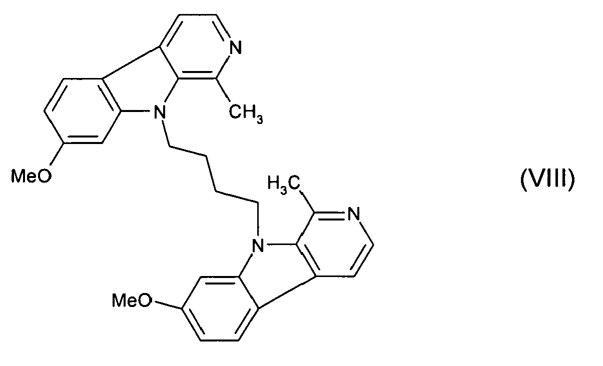
- 7,7'-[ethane-1,2-diylbis(oxyethane-2,1-diyloxy)]bis(1-methyl-9H-β-carboline) of formula (IX) 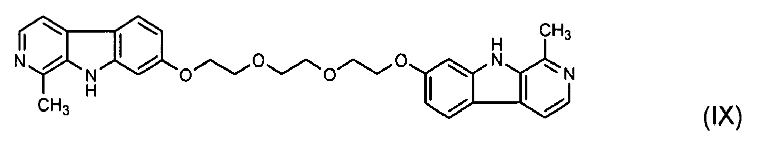
- 7,7'-[Pentane-1,5-diylbis(oxy)]bis(1-methyl-9-(4-fluorobenzyl)-9H-β-carboline) of formula (X)

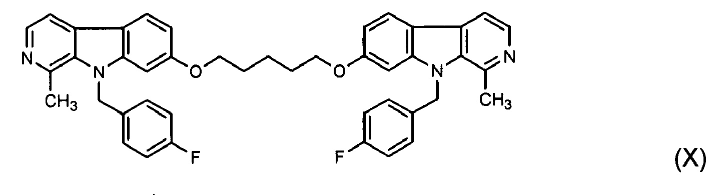

The invention also relates to processes for preparing the dimers as mentioned above.

Accordingly, the invention provides a process for the preparation of the dimers of formula (V-1), and its pharmaceutically acceptable salts which comprises:
(i) reacting the compound of formula (XI) 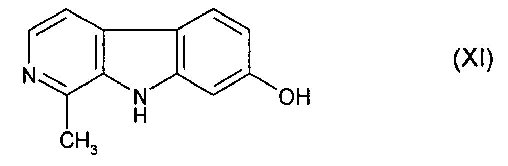 with a reactant selected from the group consisting of K₂CO₃/DMF,Cs₂CO₃/DMF, NaH/THF, and sodium ethoxide, said reactant being preferably sodium ethoxide,
   in particular in refluxing ethanol, to obtain a phenate of formula (XII): 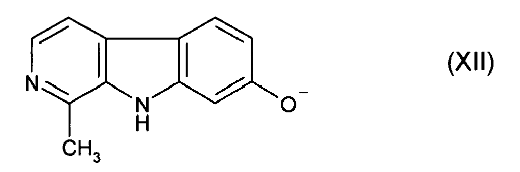
(ii) and reacting the phenate of formula (XII) with a compound of formula (XIII): L-(CH₂)ₙ-L, L representing a leaving group selected in particular from the group consisting of tosylate (OTs), mesylate (OMs), and halogen, in particular I or Br, n being as defined above in formula (V-1).

The invention also provides a process for the preparation of the compound of formula (V), and its pharmaceutically acceptable salts, which comprises the reaction of the phenate of formula (XII) with a compound of formula Hal-(CH₂)₃-Hal, Hal representing a halogen atom, in particular Br.

Thus, the invention provides a process for the preparation of the harmol dimers of formula (V) and its pharmaceutically acceptable salts, which comprises:
(i) reacting the compound of formula (XI) with sodium ethoxide in refluxing ethanol to get the sodium phenate of formula (XII), and
(ii) reacting said sodium phenate (XII) with 1,3-dibromopropane.

The invention also provides a process for the preparation of the compound of formula (VI), and its pharmaceutically acceptable salts, which comprises the reaction of the phenate of formula (XII) with a compound of formula Hal-(CH₂)₅-Hal, Hal representing a halogen atom, in particular Br.

Thus, the invention provides a process for the preparation of the harmol dimers of formula (VI) and its pharmaceutically acceptable salts which comprises:
(i) reacting the compound of formula (XI) with sodium ethoxide in refluxing ethanol to get the sodium phenate of formula (XII), and
(ii) reacting the sodium phenate with 1,5-dibromopentane.

The invention also provides a process for the preparation of the harmol dimers of formula (VII) and its pharmaceutically acceptable salts which comprises the alkylation of the compound of formula (XI) with 4,5-bis-bromomethyl-2,2-dimethyl-[1,3]dioxolane, using in particular K₂CO₃ as base in DMF.

The invention also provides a process for the preparation of a compound of formula (VIII-1), and its pharmaceutically acceptable salts, which comprises the reaction of the compound of formula (I) 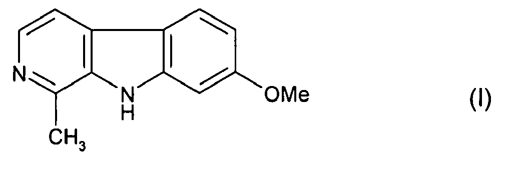 with a strong base in particular selected from the group consisting of: KH, iPr₂NLi, potassium hexamethyldisilazane (KHMDS), and NaH, said base being preferably NaH, and a compound of formula (XIV): L-(CH₂)ₙ-L, L representing a leaving group selected in particular from the group consisting of tosylate (OTs), mesylate (OMs), and halogen, in particular I or Br, and n varying from 2 to 10, preferably from 3 to 5.

Thus, the invention provides a process for the preparation of the harmol dimers of formula (VIII) and its pharmaceutically acceptable salts which comprises reacting the compound of formula (I) with NaH, and 1,5-dibromopentane.

The invention provides a process for the preparation of the harmol dimers of formula (IX) and its pharmaceutically acceptable salts which comprises:
(i) reacting the compound of formula (XI) with sodium ethoxide in refluxing ethanol to get the sodium phenate of formula (XII), and
(ii) reacting the sodium phenate with tri(ethyleneglycol)di-p-tosylate.

The invention also provides a process for the preparation of the harmol dimers of formula (X) and its pharmaceutically acceptable salts which comprises reacting the compound of formula (VI) with 4-fluorobenzyl bromide.

This process can also be carried out by reacting the compound of formula (VI) with activated benzyl halides (Ar-CH₂-Hal), or allyl derivatives, or progargyl, and derivatives thereof.

The above harmol dimers induce remodeling of the actin cytoskeleton in tumor cells, thereby leading to decreased cell motility and recovery of cell adhesion. This process leads *in vivo* to selective apoptosis of tumor cells resulting from various mechanisms including eventually from an immune response of the host possibly involving TCL toxic effect.

Thus, the invention relates to the use of a dimer as defined above, and in particular of formula (II) to (X) for the manufacture of a medicament intended for the treatment of cancer. The invention also relates to a method of treating cancer, by reversing the transformed phenotype of a tumor cell, comprising administering to a subject in need thereof a therapeutically effective amount of a harmol dimer as defined above.

The invention further relates to the use of a dimer of formula (II) to (X) for the manufacture of a medicament intended for reversing the transformed phenotype of a tumor cell. The invention also relates to a method of reversing the transformed phenotype of a tumor cell, comprising administering to a subject in need thereof a therapeutically effective amount of an harmol dimer as defined above.

As used herein, the term "subject" denotes a mammal, such as a rodent, a feline, a canine, and a primate. Preferably a subject according to the invention is a human.

In the context of the invention, the term "treating" or "treatment", as used herein, means reversing, alleviating, inhibiting the progress of, or preventing the disorder or condition to which such term applies, or one or more symptoms of such disorder or condition.

As used herein, the term "transformed phenotype" denotes a change which may occur (i) in cell morphology, and/or (ii) in the organization of the cytoskeleton, and/or (iii) in cell motility and/or (iv) in growth factor- or adhesion-dependent cell proliferation. Said transformed phenotype is a hallmark of tumor cells.

Examples of changes in cell morphology include cells displaying a more rounded shape, fewer cytoplasmic extensions, reduced spreading area, and reduced cell/cell contacts. A change in the organization of the cytoskeleton may be in particular a disruption of the actin cytoskeleton, which is typically associated with a concomitant reduction in the number of focal adhesions.

"Reversing the transformed phenotype of tumor cells" means making the tumor cells to recover the phenotype of a normal (i.e. non-tumoral) cell. Reversal of the transformed phenotype by harmol dimers is in particular induced by actin network rearrangement.

Reversal of the transformed phenotype may be assessed by the one skilled using methods of assay readily known in the art.

These methods include for instance:
- a semi-solid or methylcellulose growth assay (clonogenicity assay)
- a cell motility assay
- a method of measurement of stationary polymerized actin in a cellular lysate, as described in the international patent application WO 2004/057337. This method comprises of an index of tumor aggressivity. Briefly the method comprises lysing cells under non denaturing conditions, adjusting the total proteins concentration of the lysate, adding fluorescently labelled actin monomers and components necessary for polymerization of endogenous actin (e.g. ATP), and measuring polymerised actin;
- an assessment of morphological changes and cytoskeleton organisation of cells by actin, zyxin, actinin, or β-catenin labelling following microscopy observation according to conventional procedures.

The medicament or method according to the invention induces selective apoptosis of tumor cells and thereby provides a non-cytotoxic method of treatment of cancer.

According to the invention, the tumor cell may be a cell originating from any tumor, e.g. a primary or metastatic tumor, a solid tumor or soft tissue tumor, or a leukemia. Examples of solid or soft tumor cells include bladder, breast, bone, brain, cervical, colorectal, endometrial, kidney, liver, lung, nervous system, ovarian, prostate, testicular, thyroid, uterus, and skin cancer cells. Leukaemias include for instance chronic myeloproliferative diseases, myelodysplastic syndromes, acute non lymphocytic leukaemias, B-cell acute lymphocytic leukaemias, T-cell acute lymphocytic leukaemias, non Hodgkin lymphomas, and chronic lymphoproliferative diseases.

Tumor cells which are expected to be most responsive to the harmol dimers are those characterized by an invasive phenotype associated with cytoskeleton breakdown, increased cell motility and/or decreased cell-cell adhesion, as may be observed in aggressive sarcoma and during epithelium-mesenchymal transition occurring in early step of metastasis.

It is an advantage of the invention that pursuant to their capacity to reverse the malignant phenotype of a cell, the harmol dimers as described herein constitute true anti-invasive agents. Hence, according to an embodiment, the tumor cell is a metastatic cell. Accordingly, the medicament or method according to the invention may be intended for the treatment of metastasis.

Furthermore, the harmol dimers as defined herein have anticancer activity which is mediated by a non cytotoxic process. These compounds may advantageously be administered to treat cancer in a subject escaping conventional cytotoxic chemotherapies with inhibitors of DNA replication such as DNA binding agents in particular alkylating or intercalating drugs, antimetabolite agents such as DNA polymerase inhibitors, or topoisomerase I or II inhibitors, or with anti-mitogenic agents such as alkaloids. These cytotoxic compounds include for instance actinomycin D, adriamycin, bleomycine, carboplatin, cisplatin, chlorambucil, cyclophosphamide, doxorubicin, etoposide, 5-fluorouracil, 6-mercaptopurine melphalan, methotrexate, paclitaxel, taxotere, vinblastine, and vincristine.

As used herein, the term "subject escaping cytotoxic chemotherapy" denotes in particular subjects in which cytotoxic chemotherapy does not modify tumor progression.

One or more harmol dimers, as defined herein, may be administered simultaneously or consecutively to the subject to be treated.

Moreover, the harmol dimers may be administered in combination (i.e. simultaneously or consecutively) with a differentiating agent, in particular with vitamin A, its synthetic analogues, and metabolites (retinoids), vitamin D or its analogues, or peroxisome proliferator-activated receptors (PPAR) ligands.

Retinoids may be for instance all-transretinoic acid (ATRA), N-(4-hydroxyphenyl) retinamide (4HPR), 13-cis-retinoic acid (13-CRA), or 9-cis-retinoic acid (9-CRA).

Vitamin D or its analogues include in particular 25-dihydroxyvitamin D3 (1,25-(OH)2 D3), which is the dihydroxylated metabolite normally formed from vitamin D3, or 1alpha.-hydroxy-vitamin D3, 1alpha.-hydroxyvitamin D2, 1alpha-hydroxyvitamin D5, fluorinated vitamin D derivatives.

PPAR ligands are in particular PPARα or PPARγ activators. Selective PPARγ agonists include classic TZDs (troglitazone, rosiglitazone, pioglitazone, and ciglitizone; see Forman et al., 1995, Cell, 83:803-812; Lehmann et al., 1995, J. Biol. Chem. 270:12953 -12956) and non-TZD-type agonists. Representatives of the latter include N-(2-benzoylphenyl)-L-tyrosine derivatives, such as GW 1929, GI 262570, and GW 7845, which are among the most potent and selective PPARγ agonists identified to date (see Henke et al., 1998, J. Med. Chem., 41:5020-5036; Cobb et al., 1998, J. Med. Chem., 41:5055 -5069). GW 0207, a 2,3-disubstituted indole-5-carboxylic acid, is also a potent and selective PPARγ agonist (Henke et al., 1999, Bioorg. Med. Chem. Lett., 9:3329-3334). Fibrates or farnesol are examples of PPARα agonists.

Therefore, the harmol dimers useful according to the invention may also be mixed with another therapeutic compound to form pharmaceutical compositions (with or without diluent or carrier) which, when administered, provide simultaneous administration of a combination of active ingredients resulting in the combination therapy of the invention. In particular the invention provides a pharmaceutical composition comprising an harmol dimer of formula (II) to (X) and a differentiating agent, as are defined above.

Further to a simultaneous administration, the harmol dimers useful according to the invention may also be administered separately or sequentially with another therapeutic compound, in particular a differentiating agent as defined above. Thus the invention further provides a product comprising an harmol dimer of formula (II) to (X), and a differentiating agent, as a combined preparation for simultaneous, use for reversing the transformed phenotype of a tumor cell.

While it is possible for the harmol dimers to be administered alone it is preferably to present them as pharmaceutical compositions. The pharmaceutical compositions, both for veterinary and for human use, useful according to the present invention comprise at least one harmol dimer, as above defined, together with one or more pharmaceutically acceptable carriers and optionally other therapeutic ingredients.

In certain preferred embodiments, active ingredients necessary in combination therapy may be combined in a single pharmaceutical composition for simultaneous administration.

As used herein, the term "pharmaceutically acceptable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically such compositions are prepared as injectables either as liquid solutions or suspensions; however, solid forms suitable for solution, or suspensions, in liquid prior to use can also be prepared. The preparation can also be emulsified. In particular, the pharmaceutical compositions may be formulated in solid dosage form, for example capsules, tablets, pills, powders, dragees or granules.

The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

The pharmaceutical compositions can be administered in a suitable formulation to humans and animals by topical or systemic administration, including oral, rectal, nasal, buccal, ocular, sublingual, transdermal, rectal, topical, vaginal, parenteral (including subcutaneous, intra-arterial, intramuscular, intravenous, intradermal, intrathecal and epidural), intracisternal and intraperitoneal. It will be appreciated that the preferred route may vary with for example the condition of the recipient.

The formulations can be prepared in unit dosage form by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Total daily dose of the harmol dimers administered to a subject in single or divided doses may be in amounts, for example, of from about 0.001 to about 100 mg/kg body weight daily and preferably 0.01 to 10 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

The invention will be further illustrated in view of the following examples.

### FIGURES

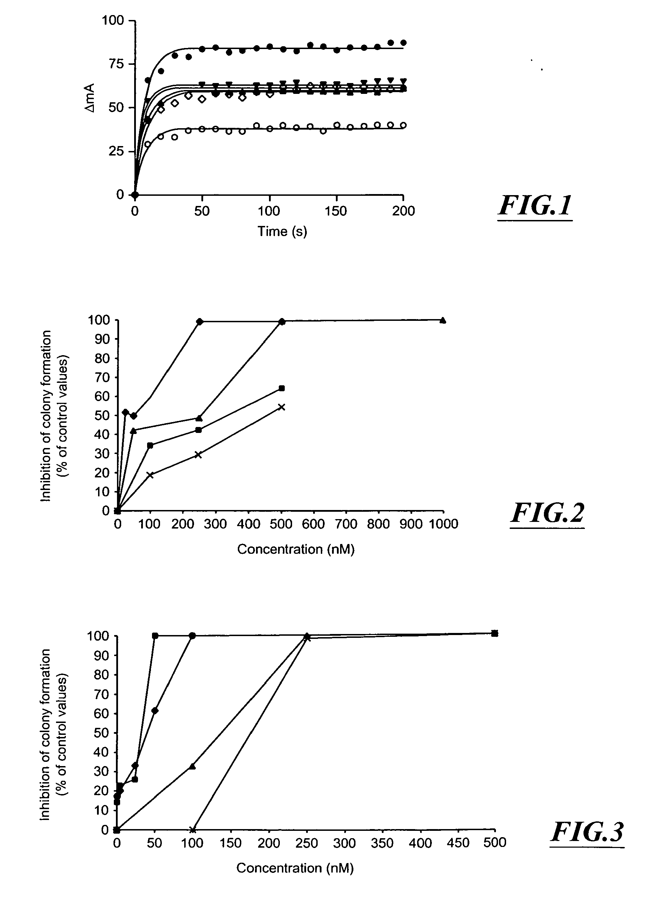

Figure 1 represents the time course of actin polymerization in the presence of harmol dimers in NIH 3T3 EF extract. Harmol dimer was added at zero time with polymerization buffer and NIH 3T3 EF extract. Reaction mixtures contained 10 µM of different harmol dimers symbolized as follows: BA016DD1172 harmol dimer (7,7'-[Pentane-1,5-diylbis(oxy)]bis(1-methyl-9H-β-carboline) - compound of formula (VI)) (▼); BA016DD1183 harmol dimer (7,7'-[(2,2-dimethyl-1,3-dioxolane-4,5-diyl)bis(methyleneoxy)]bis(1-methyl-9H-β-carboline) - compound of formula (VII) (◆); BA016FZ518 harmol dimer (9,9'-pentane-1,5-diylbis(7-methoxy-1-methyl-9H-β-carboline) - compound of formula (VIII) (0), BA016FZ527 harmol dimer (7,7'-[Propane-1,3-diylbis(oxy)]bis(1-methyl-9H-β-carboline) - compound of formula (V) (•), control malignant NIH 3T3 EF cells (○), control normal NIH 3T3 cells (▲). Data represent mean standard deviation; n = 3.

Figure 2 represents inhibition of NIH 3T3 EF colony formation in semi solid medium by BA016DD1183, BA016DD1172, BA016FZ518, and BA016FZ527.

Figure 3 represents inhibition of B16F10 colony formation in semi solid medium by BA016DD1183, BA016DD1172, BA016FZ518, and BA016FZ527.

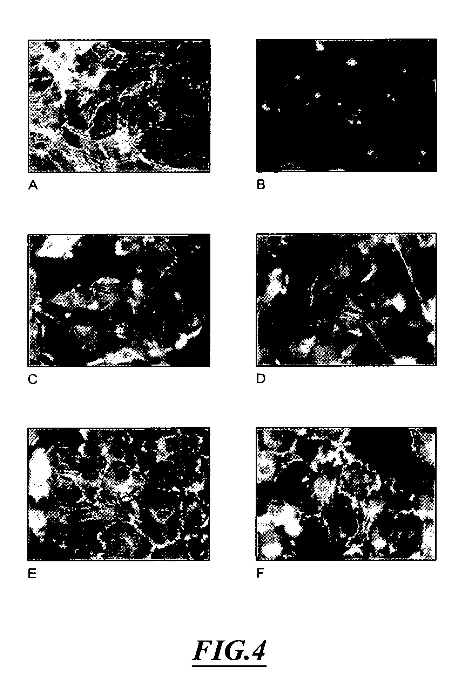

Figure 4 is a fluorescence microscopy examination of actin fibers in NIH 3T3 EF cells, treated or not with the different harmol dimers, and compared with control normal NIH 3T3 cells. Normal and malignant NIH 3T3 cells were analyzed using FITC-phalloidin to visualize the actin filaments and DAPI to visualize the nucleus. (A) Control normal NIH 3T3 cells; (B) Control malignant NIH 3T3 EF cells; (C) Malignant NIH 3T3 EF cells treated with 100 nM harmol dimer BA016DD1183; (D) Malignant NIH 3T3 EF cells treated with 100 nM harmol dimer BA016DD1172; (E) Malignant NIH 3T3 EF cells treated with 100 nM harmol dimer BA016FZ527 and (F) Malignant NIH 3T3 EF cells treated with 100 nM harmol dimer BA016FZ518.

Figure 5 represents wound healing assay performed on control NIH 3T3 EF and NIH 3T3 EF cells treated by the different harmol dimers after 10 h of cell migration into a 1 mm wound at the surface of a 6-well culture plate.

Phase contrast photographs of NIH 3T3 EF at t = 0 (A), control NIH 3T3 EF cells 10 h after wound formation (B), treated NIH 3T3 EF cells by 100 nM BA016DD1183 10 h after wound formation (C), treated NIH 3T3 EF cells by 25 nM BA016DD1172 10 h after wound formation (D), treated NIH 3T3 EF cells by 50 nM BA016FZ527 10 h after wound formation (E), and treated NIH 3T3 EF cells by 50 nM BA016FZ518 10 h after wound formation (F).

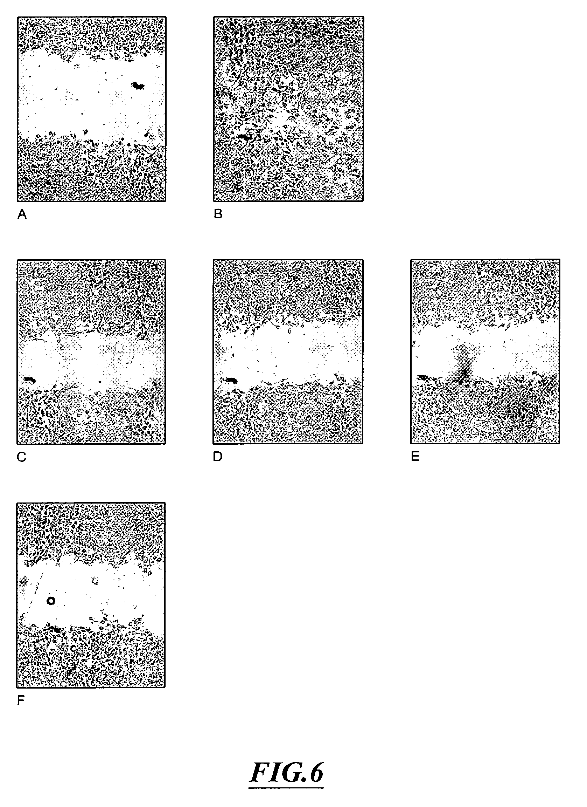

Figure 6 represents wound healing assay performed on control B16F10 and B16F10 cells treated by the different harmol dimers after 24 h of cell migration into a 1mm wound at the surface of a 6-well culture plate.

Phase contrast photographs of B16F10 at t = 0 (A), control B16F10 cells 24 h after wound formation (B), treated B16F10 cells by 100 nM BA016DD1183 24 h after wound formation (C), treated B16F10 cells by 25 nM BA016DD1172 24 h after wound formation (D), treated B16F10 cells by 100 nM BA016FZ527 24 h after wound formation (E), and treated B16F10 cells by 25 nM BA016FZ518 24 h after wound formation (F).

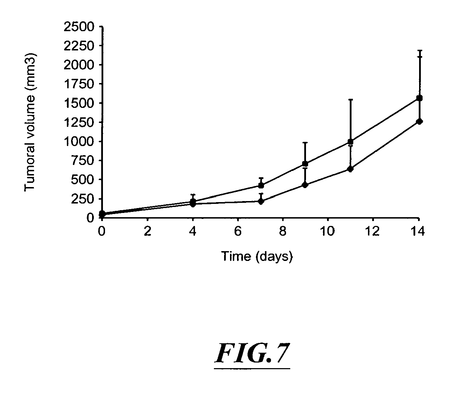

Figure 7 represents subcutaneous B16F10 tumor growth monitoring of female C57BU6 mice treated or not by BA016DD1172 harmol dimer. Control group (■); mice treated by 100 mg/Kg of harmine during 5 days (◆).

### EXAMPLES

### Example 1: Synthesis of BA016DD1172 (compound of formula (VI))

To a solution of sodium ethoxide (265 mg, 3.91 mmol) in ethanol (prepared by adding 90 mg of sodium into 6 mL of ethanol) was added in one portion 600 mg of harmol (3.03 mmol). After being stirred 5 min at 20 °C, the dibromoalkyl or bis-tosylate derivative (1.5 mmol) was added to the homogeneous solution. The reaction mixture was heated at 80 °C for 6 h. The flask was cooled to 0 °C, and the solid formed was filtered on a sintered glass funnel, and sequentially washed with a small amount of cold ethanol and diethyl ether. The crude product was taken up into water (4 mL) and heated to boiling five minutes. The solid was filtered, washed with a small amount of acetone, diethyl ether and dried under reduced pressure. When necessary, the crude mixture was purified by chromatography over silica gel eluting with CHCl₃/MeOH/NH₄OH, 100:15:2.

This process using 1,5-dibromopentane gave harmol dimerBA016DD1172 in 49 % yield. Mp = 240 - 245 °C (dec); IR (neat): n = 3500 - 2900 (broad), 2928, 2860, 1626, 1573, 1436, 1054, 1025 cm⁻¹; ¹H NMR (DMSO-*d₆*, 300 MHz): δ = 11.40 (s, 2 H), 8.14 (d, *J* = 5.0 Hz, 2 H), 8.03 (d, *J* = 8.5 Hz, 2 H), 7.79 (d, *J* = 5.0 Hz, 2 H), 7.02 (d, *J* = 2.0 Hz, 2 H), 6.84 (dd, *J* = 8.5, 2.0 Hz, 2 H), 4.12 (t, *J* = 6.2 Hz, 4 H), 2.71 (s, 6 H), 1.95 - 1.90 (m, 4 H), 1.85 - 1.80 (m, 2 H); ¹³C NMR (DMSO-*d₆*, 75 MHz): *d* = 159.4 (2 C), 141.9 (2 C), 141.1 (2 C), 137.6 (2 CH), 134.5 (2 C), 127.2 (2 C), 122.5 (2 CH), 114.7 (2 C), 111.8 (2 CH), 109.4 (2 CH), 95.2 (2 CH), 67.6 (2 CH₂), 28.5 (2 CH₂), 22.4 (CH₂), 20.0 (2 CH₃); MS (ESI+, MeOH/DMSO): m/z (%): 487 (35) [M + Na]⁺, 465 (100) [M + H]⁺; Anal. C₂₉H₂₈N₄O₂ (464.5): calcd. C 74.98, H 6.08, N 12.06; found C 74.79, H 6.23, N 11.92.

### Example 2: Synthesis of BA016DD1183 (compound of formula (VII))

A mixture of harmol (364 mg, 1.84 mmol), 4,5-bis-bromomethyl-2,2-dimethyl-[1,3]dioxolane (5) (397 mg, 1.38 mmol), K₂CO₃ (508 mg, 3.68 mmol), in DMF (4 mL) and acetone (6 mL) was heated at 60 °C. Further amounts of 5 (397 mg, 1.38 mmol) and K₂CO₃ (508 mg, 3.68 mmol), were added after 24 h and 48 h, and the heating was pursued for 3 days. After cooling, the reaction mixture was concentrated under reduced pressure. The residue was taken up into water (5 mL) and the mixture was extracted with dichloromethane (5 x 10 mL). The combined organic phases were dried over magnesium sulfate and concentrated under reduced pressure. The crude product was purified by silica gel chromatography (CHCl₃/MeOH/NH₄OH: 100:15:2) to furnish 75 mg (16% yield) of dimer 8 as a white solid. Mp = 178 - 179 °C (dec); IR (neat): ν = 3400 - 2800 (broad), 2921, 2871, 1628, 1568, 1484, 1447, 1181 cm⁻¹; ¹H NMR (DMSO-d₆, 300 MHz): δ = 11.47 (s, 2 H), 8.13 (d, J = 5.4 Hz, 2 H), 8.05 (d, J = 8.7 Hz, 2 H), 7.80 (d, J = 5.4 Hz, 2 H), 7.07 (d, J = 1.8 Hz, 2 H), 6.89 (dd, J = 8.7, 1.8 Hz, 2 H), 4.50 - 4.25 (m, 6 H), 2.71 (s, 6 H), 1.45 (s, 6 H), ¹³C NMR (DMSO-d₆, 75 MHz): δ 159.0 (2 C), 141.8 (2 C), 141.3 (2 C), 137.7 (2 CH), 134.6 (2 C), 127.1 (2 C), 122.6 (2 CH), 115.2 (2 C), 111.9 (2 CH), 109.5 (C), 109.1 (2 CH), 95.6 (2 CH), 76.0 (2 CH), 68.4 (2 CH₂), 26.9 (2 CH₃), 20.3 (2 CH₃); MS (ESI-, MeOH/DMSO): m/z (%): 521 (100) [M - H]-.

### Example 3: Synthesis of BA016FZ527 (compound of formula (V))

To a solution of sodium ethoxide (265 mg, 3.91 mmol) in ethanol (prepared by adding 90 mg of sodium into 6 mL of ethanol) was added in one portion 600 mg of harmol (3.03 mmol). After being stirred 5 min at 20 °C, the dibromoalkyl or bis-tosylate derivative (1.5 mmol) was added to the homogeneous solution. The reaction mixture was heated at 80 °C for 6 h. The flask was cooled to 0 °C, and the solid formed was filtered on a sintered glass funnel, and sequentially washed with a small amount of cold ethanol and diethyl ether. The crude product was taken up into water (4 mL) and heated to boiling five minutes. The solid was filtered, washed with a small amount of acetone, diethyl ether and dried under reduced pressure. When necessary, the crude mixture was purified by chromatography over silica gel eluting with CHCl₃/MeOH/NH₄OH, 100:15:2.

This process using 1,3-dibromopropane gave harmol dimer BA016FZ527 in 35 % yield. Mp = 280 - 282 °C (dec); IR (neat): n = 3400-2700 (broad), 3071, 2965, 2876, 1627, 1568, 1486, 1446 cm⁻¹; ¹H NMR (DMSO-*d₆*, 200 MHz): δ = 11.30 (s, 2 H), 8.14 (d, *J* = 5.4 Hz, 2 H), 8.05 (d, *J* = 8.7 Hz, 2 H), 7.79 (d, *J* = 5.4 Hz, 2 H), 7.06 (d, *J* = 2.0 Hz, 2 H), 6.90 (dd, *J* = 8.7, 2.0 Hz, 2 H), 4.32 (t, *J* = 6.2 Hz, 4 H), 2.71 (s, 6 H), 2.33 (m, 2 H); ¹³C NMR (DMSO-*d₆*, 50 MHz): d = 159.2 (2 C), 141.8 (2 C), 141.2 (2 C), 137.7 (2 CH), 134.5 (2 C), 127.2 (2 C), 122.6 (2 CH), 114.9 (2 C), 111.8 (2 CH), 109.3 (2 CH), 95.4 (2 CH), 64.6 (2 CH₂), 28.7 (CH₂), 20.2 (2 CH₃); MS (ESI+, MeOH/DMSO): m/z (%): 459 (90) [M + Na]⁺, 437 (100) [M + H]⁺, 359 (85); Anal. C₂₇H₂₄N₄O₂.5/4H₂O (459.0): calcd. C 70.65 , H 5.82, N 12.21; found C 70.87, H 5.62, N 12.19.

### Example 4: Synthesis of BA016FZ518 (compound of formula (VIII))

To a suspension of sodium hydride (115 mg, 60% in mineral oil, 2.8 mmol) in DMF (4 mL) was added harmine 12 (500 mg, 2.35 mmol). When the vigorous hydrogen evolution was over, 1,5-dibromopentane (230 mg, 1.0 mmol) was added. The resulting mixture was stirred at 25 °C for 24 h. Water was added and the mixture was extracted with dichloromethane (3 x 15 mL). The combined organic phases were dried over magnesium sulfate and concentrated under reduced pressure. The residue was purified by chromatography on silica gel eluting with ethyl acetate to give dimer 14 as a white solid (360 mg, 74% yield). Mp = 168 -169 °C; IR (neat) ν = 2989, 2926, 1620, 1561, 1441, 1402, 1340, 1251, 1203 cm⁻¹; ¹H NMR (CDCl₃, 300 MHz) δ 8.27 (d, J = 5.4 Hz, 2 H), 7.96 (d, J = 8.5 Hz, 2 H), 7.72 (d, J = 5.4 Hz, 2 H), 6.88 (dd, J = 8.5, 2.1 Hz, 2 H), 6.74 (d, J = 2.1 Hz, 2 H), 4.41 (t, J = 7.5 Hz, 4 H), 3.87 (s, 6 H), 2.95 (s, 6 H), 1.88 - 1.80 (quint, J = 7.5 Hz, 4 H), 1.45 - 1.40 (m, 2 H); ¹³C NMR (CDCl3, 75 MHz): δ 160.9 (2 C), 143.1 (2 C), 140.3 (2 C), 138.2 (2 CH), 135.2 (2 C), 129.6 (2 C), 122.5 (2 CH), 115.2 (2 C), 112.3 (2 CH), 108.7 (2 CH), 93.5 (2 CH), 55.7 (2 CH₃), 44.6 (2 CH₂), 30.6 (2 CH₂), 24.4 (CH₂), 23.3 (2 CH₃); MS (ESI+, MeOH/DMSO): m/z (%): 515 (15) [M + Na]⁺, 493 (50) [M + H]⁺, 479 (100); Anal. C₃₁H₃₂N₄O₂. H₂O (510.6): calcd. C 72.92, H 6.71, N 10.97; found C 72.84, H 6.51, N 11.02.

### Example 5: Modulation of actin dynamics

Actin dynamics have been quantified in tumor cell cytosolic fraction using the fluorescence anisotropy assay which gains access to rate constant of F-actin elongation (kobs) and steady state concentration of F-actin (Δ mA max).

### Materials and methods

All reactions were carried out at 22°C and fluorescence anisotropy signal was recovered at 520 nm with excitation at 490 nm in a Beacon 2000 (Panvera). Alexa 488 actin (Molecular Probes) was centrifuged at 35 000 rpm for 2 h at 4°C to sediment residual actin polymers in a Beckman L5-50B ultracentrifuge. The fluorescence remaining in the supernatant was considered to be likely due to monomers or small actin filaments (5-10 monomers) that do not pellet under conditions described previously. 80% of the supernatant was withdrawn; the concentration was defined through fluorescence measurements (excitation at 490 nm and signal recovering at 520 nm). The ultracentrifuged actin concentration was calculated using the non ultracentrifuged Alexa 488 actin as a standard. The supernatant was aliquoted, frozen in liquid nitrogen and stored at -80°C.

Before experiment, an aliquot of ultracentrifuged Alexa 488 actin was diluted to a concentration of 1 mg/ml in G buffer (5 mM Tris pH 8.1, 2 mM CaCl2, 0.2 mM DTT, 0.2 mM ATP). 3 µl of diluted Alexa 488 actin was mixed in 168 µl of G buffer and actin monomers anisotropy was measured before the addition of 4 µl of polymerisation buffer (2.5 M KCI, 50 mM MgCl2, 25 mM ATP), 5 µl of G buffer in the presence or in the absence of the chemical molecule and 20 µl cellular extract of normal NIH 3T3 cells or malignant NIH 3T3 EF cells at 2 mg/ml. The final concentration of Alexa 488 actin was 4 nM. The ratio of unlabelled to labelled actin was about 140/4 nM. Measurements were made each 10 sec for 200 sec. Actin monomers anisotropy value was subtracted, yielding the anisotropy enhancement (Δ mA). The data were fitted with the equation Y= Ymax . [1- exp(- K.X)]. The curves start at zero and ascend to Ymax that corresponds to the steady state anisotropy value (Δ mA eq), with a rate constant K. Y is anisotropy values of which monomers anisotropy is substracted and X is the time (Assessment of cellular actin dynamics by measurement of fluorescence anisotropy. Anal Biochem. 2007 Aug 1;367(1):95-103. Epub 2007 Apr 5. Spitz et al.).

### Results

The effects of BA016DD1172, BA016DD1183, BA016FZ527, and BA016FZ518 on these parameters were as follows:

**Table 1: Effects of 10 µM of the different harmol dimers on anisotropy enhancement in NIH 3T3 EF extracts.**

| | NIH 3T3 | NIH 3T3 EF | EF + 10 µM BA016DD1172 | EF + 10 µM BA016DD1183 | EF + 10 µM BA016 FZ518 | EF + 10 µM BA016 FZ527 |
|---|---|---|---|---|---|---|
| Kobs | 0,122 | 0,120 | 0,179 | 0,158 | 0,104 | 0,132 |
| Δ mA max | 59 | 38 | 62 | 61 | 59 | 83 |

When added to assay medium, harmol dimers BA016DD1172, BA016DD1183, BA016FZ527, and BA016FZ518 increased the F actin elongation rate constant (Kobs) and the F actin steady state value (Δ mA max). Figure 1 showed typical kinetics observed following the addition of these different harmol dimers. In the presence of 10 µM of BA016DD1172, BA016DD1183, and BA016FZ518, the actin polymerisation kinetics of the tumoral NIH 3T3 EF cytosolic fractions was similar to those observed using normal NIH 3T3 cytosolic fraction.

Moreover, in the presence of 10 µM BA016FZ527, the F actin elongation rate constant and the F actin steady state value were higher to those observed in NIH 3T3 cytosolic fraction.

### Example 6: Ex vivo antiproliferative effect

### Material and methods:

### - MTT assay

Growth studies were performed using the [3-(4,5 dimethylthiazol-2-yl)-2,5-diphenyl-2H-tetrazolium bromide] (Sigma) colorimetric assay.

About 1500 cells were seeded in a 96-well culture plate 24 hours before adding increasing concentrations of tested molecule. The plate was incubated at 37°C for 3 days. 10 µl MTT stock solution (5 mg/ml in phosphate buffer saline) was added to 90 µl of complete medium in each well, the incubation was continued for 3 h at 37°C. 100 µl of lysis buffer (10 % sodium dodecyl sulfate, 1% HCl 1N; pH 4.7) was added to each well, and the plate was incubated overnight. The absorbance was determined using an Integrated EIA Management System (Labsystem) at a wavelength of 570 nm. The proliferation rates were calculated from the OD readings using the untreated cells as 100%.

### - Cloning assay

Cells were embedded in complete culture medium supplemented with 0,8% methylcellulose (Methocel MC4000, Sigma), seeded in triplicate into 35-mm dishes (Greiner Bio-one Ref 627102, Dominique Dutscher) and incubated at 37 °C in a humidified 5% CO₂ atmosphere.

The number of cells seeded was 1000 cells per dish. After one week (B16F10 cell line) to 3 weeks (NIH 3T3 EF cell line), macroscopic clones were counted.

### Results

Inhibition of colony formation occurred at non-cytotoxic concentrations as measured using the MTT test.

**Table 3: effect of BA016DD1172 on NIH 3T3 EF and B16F10 cellular viability and inhibition of colony formation in semi solid medium (methylcellulose).**

| | Cellular viability (MTT) | Inhibition of colony formation (methylcellulose) |
|---|---|---|
| NIH 3T3 EF | IC50 = 300 nM | IC50 = 50 nM |
| B16F10 | IC50 = 400 nM | IC50 = 30 nM |

**Table 4: effect of BA016FZ518 on NIH 3T3 EF and B16F10 cellular viability and inhibition of colony formation in semi solid medium (methylcellulose).**

| | Cellular viability (MTT) | Inhibition of colony formation (methylcellulose) |
|---|---|---|
| NIH 3T3 EF | IC50 = 750 nM | IC50 = 250 nM |
| B16F10 | IC50 = 3 µM | IC50 = 40 nM |

**Table 5: effect of BA016FZ527 on NIH 3T3 EF and B16F10 cellular viability and inhibition of colony formation in semi solid medium (methylcellulose).**

| | Cellular viability (MTT) | Inhibition of colony formation (methylcellulose) |
|---|---|---|
| NIH 3T3 EF | IC50 = 1 µM | IC50 = 500 nM |
| B16F10 | IC50 = 1,5 µM | IC50 = 175 nM |

**Table 6: effect of BA016DD1183 on NIH 3T3 EF and B16F10 cellular viability and inhibition of colony formation in semi solid medium (methylcellulose).**

| | Cellular viability (MTT) | Inhibition of colony formation (methylcellulose) |
|---|---|---|
| NIH 3T3 EF | IC50 = 1 µM | IC50 = 300 nM |
| B16F10 | IC50 = 750 nM | IC50 = 125 nM |

BA016DD1172, BA016DD1183, BA016FZ527, and BA016FZ518 were found to display a marked inhibitory activity on NIH 3T3 EF (Figure 2), and B16F10 (Figure 3) colony formation in methylcellulose semi-solid medium

### Example 7: Rescue of F-actin network in tumor cells

### Material and methods

Malignant NIH 3T3 EF cells were seeded onto glass cover slips at a density of 2000 cells per cm². The next day, tested molecules were applied to NIH 3T3 EF cells at various non cytotoxic concentrations. Three days later, cells were fixed for 10 min in PBS containing 3.7 % formaldehyde at 4°C before examination with a fluorescence microscope. The formaldehyde solution was neutralized with 50 mM NH4Cl. Permeabilisation was carried out for 4 min with 0.4% Triton X-100 in PBS. Cells were incubated for 1 h at room temperature with blocking buffer (3% bovine serum albumin in PBS) and then for 1 h with FITC-phalloidin (Sigma) which stabilizes structures by inhibiting phosphate release from ADP-Pi-actin filaments and allows visualization of actin filaments by fluorescence microscopy. The cover slips were mounted on microscope slides using a solution of glycerol 50%, DABCO 2.5% in PBS as embedding medium.

### Results

The drugs BA016DD1172, BA016DD1183, BA016FZ527, and BA016FZ518 were able to reorganise the actin network in tumor cells at a non cytotoxic concentration (100 nM) as shown in Figure 4. Tumoral NIH 3T3 EF cells, treated with a non cytotoxic concentration of these harmol dimers, recovered a morphology close to that of normal NIH 3T3 cell : cells was spread, present numerous intercellular contacts and actin cytoskeleton was well organized in a stress fibres network.

### Example 8: Ex vivo inhibition of cell motility

### Material and methods

Harmol dimers have been tested in a cell motility assay: the behaviour of tumoral cells treated by these dimers was compared with non-treated tumoral cells in a wound healing assay. Wound healing assay was performed to evaluate the effect of tested molecules on motility of malignant NIH 3T3 EF cells and melanoma cell line B16F10. About 100 000-200 000 cells were seeded in a 6-well culture plate and the tested molecule was added 24 hours later. Cells were grown for 3 days to confluence about 90-95% and small scratch-wounds (1 mm width) were made with a pipette tip. Cell debris were removed, then the cultures were incubated in complete medium for 10 h in the presence of the same concentration of the tested molecule. Then, the cells were fixed for 10 min in PBS containing 3.7% formaldehyde at 4°C. The healing was observed with a phase contrast light microscope using Zeiss software.

### Results

Murine melanoma cells B16F10 and tumoral NIH 3T3 EF cells displayed a high motility phenotype. The non treated tumoral cells NIH 3T3 EF and B16F10 migrate beyond the border of the wound into its whole area. At the opposite, the malignant cells treated by harmol dimers did not migrate at all into the wound.

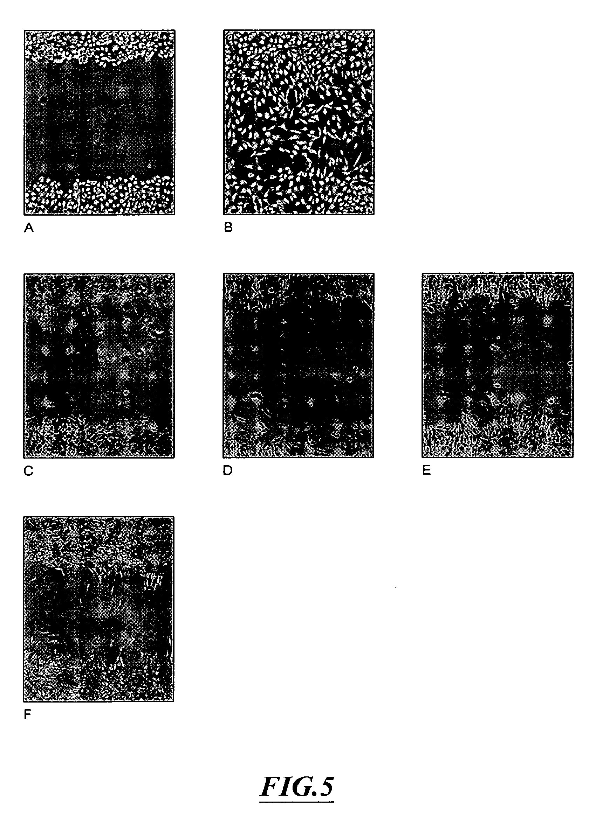

BA016DD1183, BA016DD1172, BA016FZ518 and BA016FZ527 inhibited the malignant cell motility at a non cytotoxic concentration (Figures 5 and 6).

### Example 9: In vivo tumorigenicity assay

### Material and methods

Female 6-8 week-old C57BU6 mice were provided by Harlan (Gannat, France).

Animals were housed in air-conditioned atmosphere, given food and water in standard conditions. One million B16F10 cells in 0.1 ml PBS were injected subcutaneously into the right flank of mice. Seven days after cells inoculation, it was possible to measure tumor volume (day 0). Thereafter, before drug administration, female C57BU6 mice were randomized to form 2 groups. The BA016DD1172 treatment consisted of a daily per os administration at 100 mg/kg for five days (day 0 to day 5).

B16F10 tumor development was monitored for 2 weeks. The tumor size was estimated every fourth day by measuring the diameters of the tumors in two perpendicular dimensions using callipers. The volume of tumor (V) was calculated using the formula, V = 2W(U2)2 , where L and W were respectively the length and the width of tumor.

### Results

BA016DD1172 exhibited a marked antitumor activity against B16 melanoma (Figure 7). At day 7, the tumoral growth inhibition of the treated group was 48,5% of the value observed for the control group (p = 0,0020).

### Example 10: comparison with harmine and harmol

The same experiments are done with harmine in order to compare with the efficacy of the harmol dimers.

The modulation of actin dynamics was measured in tumoral NIH3T3 EF cellular lysate incubated with harmol at 10 µM. No increase of the value of the F actin steady state was observed (Δ mA max = 42 mA).

Furthermore, harmol did not display an inhibitory activity on B16F10 colony formation in methylcellulose semi-solid medium at 3 µM (non-cytotoxic concentration as measured by MTT test) whereas harmol dimers inhibit B16F10 colony formation for nanomolar concentrations.

When harmine is added to assay medium, an increase of F actin elongation rate constant and F actin steady state value is observed as well, but the F actin elongation rate is lower than in normal NIH 3T3 cytosolic fraction and tumoral NIH 3T3 EF cytosolic fractions incubated with BA016DD1172, BA016DD1183, BA016FZ527, and BA016FZ518 dimers.

In the case of harmine an inhibition of NIH 3T3 EF and B16F10 colony formation was observed but for higher concentrations than BA016DD1172, BA016DD1183, BA016FZ527 and BA016FZ518 concentrations (micromolar concentration for harmine versus nanomolar concentrations for the others harmol dimers in the case of NIH 3T3 EF colonies; about 1 µM concentration for harmine versus nanomolar concentrations for the others harmol dimers in the case of B16F10 colonies).

When NIH 3T3 EF cells were incubated with harmine, modification in actin cytoskeleton network was observed for higher concentrations than those used in harmol dimers test.

Tumoral NIH 3T3 EF cells treated by harmine at a higher concentration (i.e, 5 µM) presented a lower inhibition of migration than those treated with BA016DD1183, BA016DD1172, BA016FZ518, and BA016FZ527.

*In vivo* tumorigenicity assay was studied with harmine. The treatment with harmine consisted in a daily per os administration at 100 mg/kg (2 x 50 mg/kg) and 200 mg/Kg (2 x 100 mg/kg) for 15 days (day 0 to day 14). In parallel, control mice were treated by one daily oral route administration of the vehicle. Any tumoral growth inhibition was observed in mice group treated by harmine with daily oral administrations at 100 mg/kg and 200 mg/kg during 15 days.

## Claims

1. A compound of formula (II):
A-X-A (II)
wherein:
• A represents:
- a radical of formula (1): 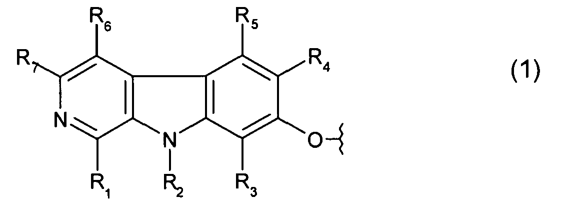
wherein:
* R₁ is chosen from the group consisting of:
. an alkyl group of 1 to 20 carbon atoms, in particular a methyl group,
. a COOR group, wherein R represents H or an alkyl group of 1 to 20 carbon atoms, and
. a COR group, wherein R represents an alkyl group of 1 to 20 carbon atoms, preferably a methyl group,
* R₂ is chosen from the group consisting of:
. H,
. an alkyl group of 1 to 20 carbon atoms,
. an aryl group of 6 to 30 carbon atoms, in particular a benzyl group, if appropriate substituted with a halogen such as F, said aryl group being preferably p-fluorobenzyl,
. a COOR group, wherein R represents H or an alkyl group of 1 to 20 carbon atoms, R being in particular a t-butyl group, and
. a COR group, wherein R represents an alkyl group of 1 to 20 carbon atoms, preferably a methyl group,
. a SO₂Ar group, wherein Ar represents an aryl group of 6 to 30 carbon atoms,
* R₃, R₅, R₆, and R₇ represent, independently from each other, a group selected from:
. H,
. an alkyl group of 1 to 20 carbon atoms, and
. an aryl group of 6 to 30 carbon atoms, in particular a benzyl group, if appropriate substituted with a halogen,
* R₄ represent, independently from each other, a group selected from:
. H,
. an alkyl group of 1 to 20 carbon atoms,
. an aryl group of 6 to 30 carbon atoms, in particular a benzyl group, if appropriate substituted with a halogen,
. NO₂, and
. a NRR' group, wherein R and R' represent, independently from each other, H or an alkyl group of 1 to 20 carbon atoms,
- or a radical of formula (2): 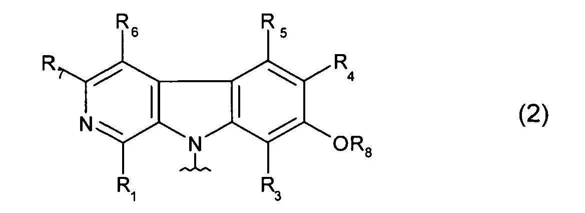
wherein:
* R₁, R₃, R₄, R₅, R₆, and R₇ are as defined above for formula (1),
* R₈ represents a group selected from:
. H,
. an alkyl group of 1 to 20 carbon atoms, and
. a COR group, wherein R represents an alkyl group of 1 to 20 carbon atoms,
- or a radical of formula (3): 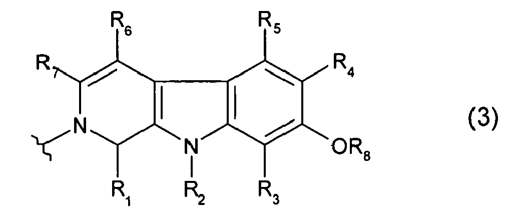
wherein R₁ to R₈ are as defined above for formula (2),
• X represents a saturated or unsaturated alkyl radical having 1 to 20 carbon atoms, eventually substituted with at least 1 oxygen atom, and preferably with at least 2 oxygen atoms, or a radical having the formula -(CH₂CH₂O)ₙCH₂CH₂-, where n is a whole number ranging from 1 to 10, said alkyl radical being eventually substituted with at least one hydroxyl group, in particular with two hydroxyl groups, eventually engaged in a 5-membered ring to form a 1,3-dioxolane cycle, which is if appropriate substituted with at least one alkyl group of 1 to 10 carbon atoms, said 5-membered ring being in particular 2,2-dimethyl-1,3-dioxolane,
or a pharmaceutically acceptable salt thereof.

2. The compound of claim 1, of formula (III): 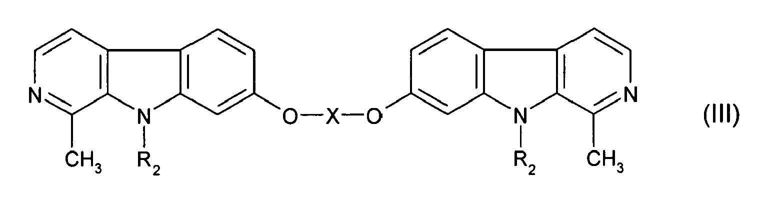 or a pharmaceutically acceptable salt thereof,
wherein:
- X is a saturated or unsaturated alkyl radical having 1 to 20, preferably 3 to 10, carbon atoms, or a radical having the formula -(CH₂CH₂O)ₙCH₂CH₂-, where n is a whole number ranging from 1 to 10,
said alkyl radical being if appropriate substituted with at least one carbonyl, or peptidic bond, or at least one oxygen atom, or with at least one hydroxyl group, in particular with two hydroxyl groups, said hydroxyl groups being eventually engaged in a 5-membered ring to form a 1,3-dioxolane cycle, which is if appropriate substituted with at least one alkyl group of 1 to 10 carbon atoms, said 5-membered ring being in particular 2,2-dimethyl-1,3-dioxolane,
R₂ is as defined in claim 1.

3. The compound of claim 2, wherein X is selected from the group consisting of the following groups:
- a group of formula (3):
-(CH₂)ₙ- (3)
n being an integer varying from 1 to 10, and preferably equal to 3, 4, 5, or 6,
- a group of formula (4):
-(CH₂)ₘ-O-(CH₂)ₚ-O-(CH₂)_{q}- (4)
m, p, and q being integers varying from 1 to 4, and preferably equal to 2,
- a group of formula (5): 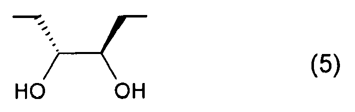 n being as defined in claim 3
- a group of formula (6):

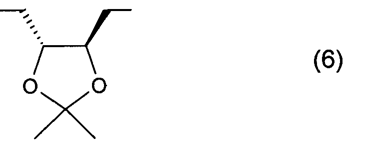

4. The compound of claim 2 or 3, or a pharmaceutically acceptable salt thereof, of one of the following formulae (V-1), (V), (VI) or (VII): 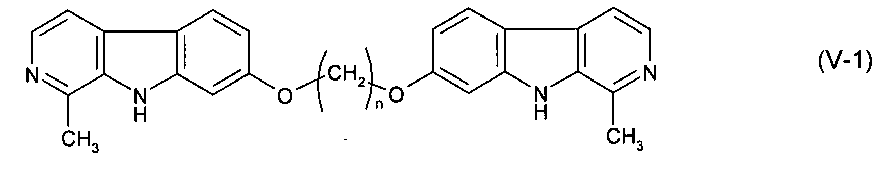 n being as defined in claim 3

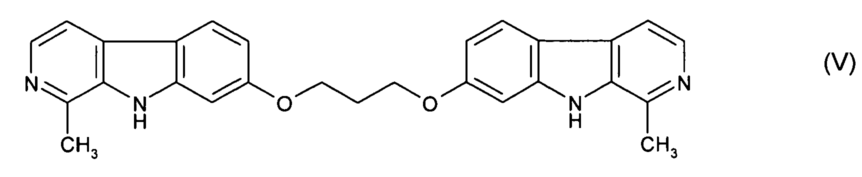

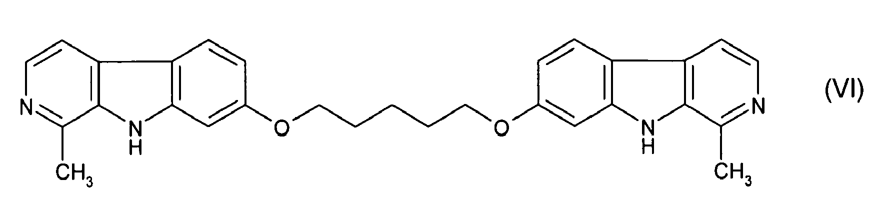

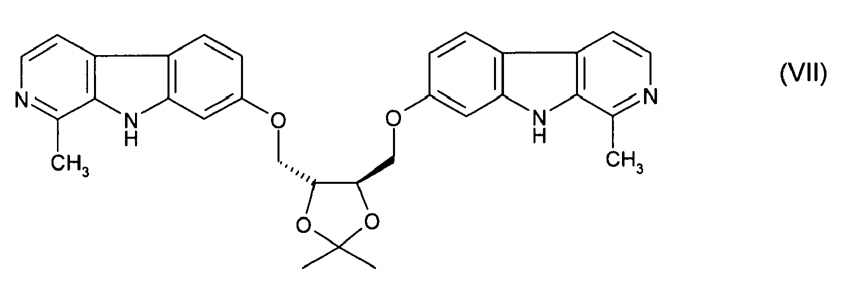

5. The compound of claim 1, of formula (IV): 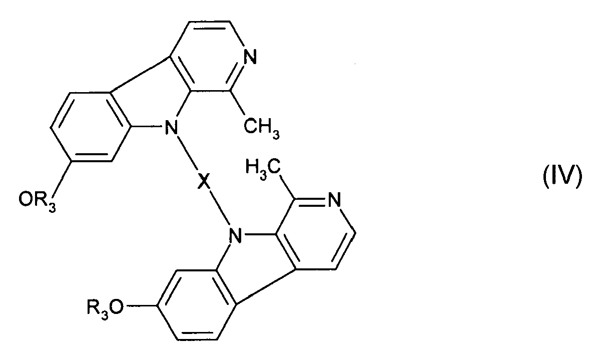 or a pharmaceutically acceptable salt thereof,
wherein:
- X is an alkyl radical having 2 to 10, preferably 3 to 5, carbon atoms, and
- R₃ is as defined in claim 1.

6. The compound of claim 5, or a pharmaceutically acceptable salt thereof, of formula (VIII-1): 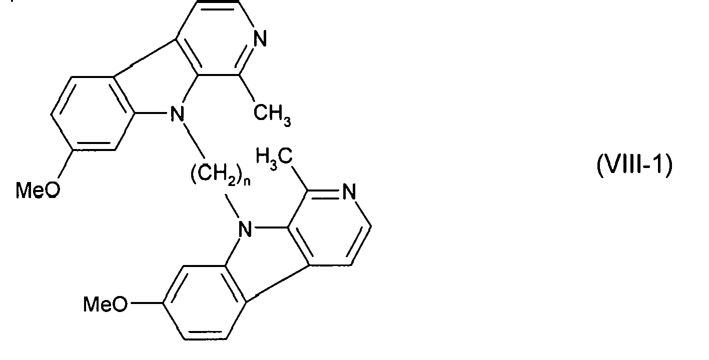 n varying from 2 to 10, preferably from 3 to 5.

7. The compound of claim 5 or 6, or a pharmaceutically acceptable salt thereof, of formula (VIII):

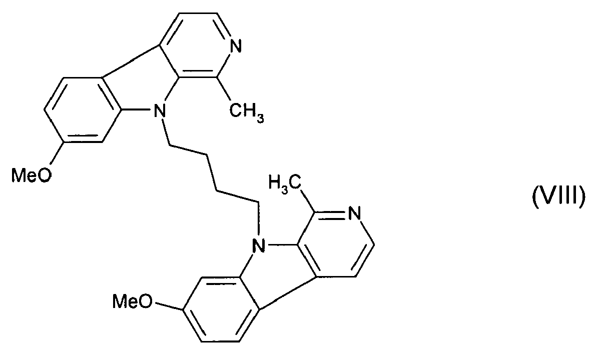

8. A pharmaceutical composition comprising a compound according to any of claims 1 to 7, in a pharmaceutically acceptable carrier.

9. The pharmaceutical composition according to claim 8, further comprising a differentiating agent.

10. The pharmaceutical composition according to claim 9, wherein said differentiating agent is selected from the group consisting of vitamin A and its synthetic analogues, retinoids, vitamin D and its analogues, and peroxisome proliferator-activated receptors (PPAR) ligands.

11. A product comprising a compound as defined in any of claims 1 to 7, and a differentiating agent, as a combined preparation for simultaneous, separate or sequential use for reversing the transformed phenotype of a tumor cell.

12. The product according to claim 11, wherein said differentiating agent is selected from the group consisting of vitamin A and its synthetic analogues, retinoids, vitamin D and its analogues, and peroxisome proliferator-activated receptors (PPAR) ligands.

13. A method for the preparation of the compound of formula (V-1) of claim 4, and its pharmaceutically acceptable salts which comprises:
(iii) reacting the compound of formula (XI) 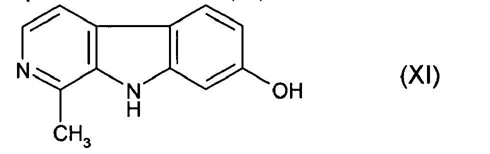 with a reactant selected from the group consisting of K₂CO₃/DMF,Cs₂CO₃/DMF, NaH/THF, and sodium ethoxide, said reactant being preferably sodium ethoxide,
in particular in refluxing ethanol, to obtain a phenate of formula (XII): 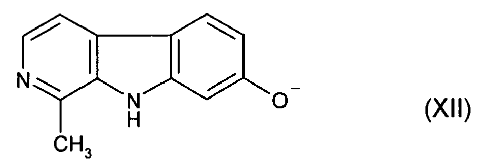
(iv) and reacting the phenate of formula (XII) with a compound of formula (XIII): L-(CH₂)ₙ-L, L representing a leaving group selected in particular from the group consisting of tosylate (OTs), mesylate (OMs), and halogen, in particular I or Br, n being as defined above in formula (V-1).

14. The method according to claim 13, for the preparation of the compound of formula (V) of claim 4, and its pharmaceutically acceptable salts, which comprises the reaction of the phenate of formula (XII) with a compound of formula Hal-(CH₂)₃-Hal, Hal representing a halogen atom, in particular Br.

15. The method according to claim 13, for the preparation of the compound of formula (VI) of claim 4, and its pharmaceutically acceptable salts, which comprises the reaction of the phenate of formula (XII) with a compound of formula Hal-(CH₂)₅-Hal, Hal representing a halogen atom, in particular Br.

16. A method for the preparation of the compound of formula (VII) of claim 4 and its pharmaceutically acceptable salts, said method comprising the alkylation of the compound of formula (XI) as defined in claim 12 with 4,5-bis-bromomethyl-2,2-dimethyl-[1,3]dioxolane, using in particular K₂CO₃ as base in DMF.

17. A method for the preparation of a compound of formula (VIII-1) of claim 6, and its pharmaceutically acceptable salts, which comprises the reaction of the compound of formula (I) 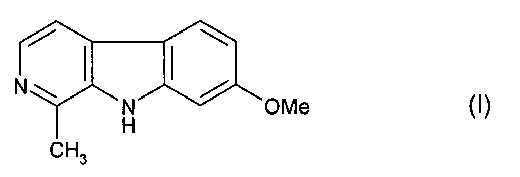 with a strong base in particular selected from the group consisting of: KH, iPr₂NLi, KHMDS, and NaH, said base being preferably NaH, and a compound of formula (XIV): L-(CH₂)ₙ-L, L representing a leaving group selected in particular from the group consisting of tosylate (OTs), mesylate (OMs), and halogen, in particular I or Br, and n varying from 2 to 10, preferably from 3 to 5.

18. A method for the preparation of the compound of formula (VIII) of claim 7, and its pharmaceutically acceptable salts, which comprises the reaction of the compound of formula (I) as defined in claim 17 with NaH and 1,5-dibromopentane.

19. The use of a compound according to any claims 1 to 7 for the preparation of a medicament intended for the prevention or treatment of cancer.

20. The use of a compound according to any of claims 1 to 7, wherein the medicament is intended for reversing the transformed phenotype of a tumor cell.

21. The use according to claims 19 or 20, wherein said tumor cell is **characterized by** an invasive phenotype.

22. The use according to any of claims 19 to 21, wherein said medicament is intended for the treatment of cancer in a subject escaping cytotoxic chemotherapy.

23. The use according to any of claims 19 to 22, wherein said medicament is administered in combination with a differentiating agent.
